(19) Europäisches Patentamt
European Patent Office
Office européen des brevets

(11) EP 0 417 698 B1

(12) **EUROPÄISCHE PATENTSCHRIFT**

(45) Veröffentlichungstag und Bekanntmachung des Hinweises auf die Patenterteilung:
**13.03.1996 Patentblatt 1996/11**

(51) Int Cl.$^6$: **C07K 5/02**, C07D 401/12, C07D 401/14, A61K 31/44

(21) Anmeldenummer: **90117400.3**

(22) Anmeldetag: **10.09.1990**

(54) **Aminosäurederivate mit reninhemmenden Eigenschaften, Verfahren zu deren Herstellung, diese enthaltende Mittel und deren Verwendung**

Amino acid derivatives having renin-inhibiting activity, process for their preparation, agents containing them, and their use

Dérivés d'acides aminés à activité inhibitrice de la rénine, procédé pour leur préparation, agents les contenant et leur utilisation

(84) Benannte Vertragsstaaten:
**AT BE CH DE DK ES FR GB GR IT LI LU NL SE**

(30) Priorität: **12.09.1989 DE 3930397**
**04.10.1989 DE 3933096**

(43) Veröffentlichungstag der Anmeldung:
**20.03.1991 Patentblatt 1991/12**

(73) Patentinhaber: **HOECHST AKTIENGESELLSCHAFT**
**D-65926 Frankfurt am Main (DE)**

(72) Erfinder:
- **Heitsch, Holger, Dr.**
  **D-6238 Hofheim am Taunus (DE)**
- **Henning, Rainer, Dr.**
  **D-6234 Hattersheim am Main (DE)**
- **Linz, Wolfgang, Dr.**
  **D-6500 Mainz (DE)**
- **Nickel, Wolf-Ulrich, Dr.**
  **D-6238 Hofheim am Taunus (DE)**
- **Ruppert, Dieter, Dr.**
  **D-6242 Kronberg/Taunus (DE)**
- **Urbach, Hansjörg, Dr.**
  **D-6242 Kronberg/Taunus (DE)**

(56) Entgegenhaltungen:
**EP-A- 0 231 919**
**EP-A- 0 239 874**
**EP-A- 0 255 082**
**EP-A- 0 310 071**
**EP-A- 0 310 072**
**EP-A- 0 329 013**
**WO-A-89/00161**

- **JOURNAL OF MEDICINAL CHEMISTRY, Band 31, 1988; J.J. PLATTNER et al., Seiten 2277-2288**

EP 0 417 698 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Aus EP-A-184 855, EP-A-189 203, EP-A-202 571, EP-A-229 667, EP-A-230 266, EP-A-237 202, EP-A-310 071, EP-A-310 072, WO-A-87/05302 und WO 88/05050 sind Aminodiol-Derivate mit reninhemmender Wirkung bekannt.

Weiterhin sind Renin-Inhibitoren in Biochem. Biophys. Res. Comm. 132, 155-161 (1985), in Biochem. Biophys. Res. Comm. 146, 959-963 (1987) in FEBS Lett. 230, 38-42 (1988), in J. Med. Chem. 30, 976 (1987) und J. Med. Chem. 31, 2277 (1988) beschrieben.

Es wurde nun gefunden, daß am N-Terminus azacyclische Acyl-aminoacyl-substituierte Aminodiolderivate mit heterocyclischer Substitution am C-Terminus überraschenderweise außergewöhnlich hochwirksame und hochspezifische Reninhemmer sind, die eine erhebliche verbesserte Resorption und wesentlich verlängerte Wirkdauer in vivo aufweisen.

Die Erfindung betrifft daher Verbindungen der Formel I

$$R^1 - X - Y - \underset{}{\overset{R^2}{CH}} - \underset{\underset{O}{\|}}{C} - B - NH - \overset{R^3}{CH} - \overset{OH}{CH} - \overset{R^4}{CH} - R^5 \qquad (I)$$

in welcher

$R^1$ einen Rest der Formel II, III oder IV

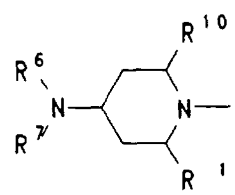
(II)

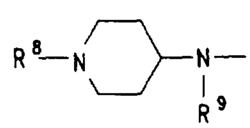
(III)

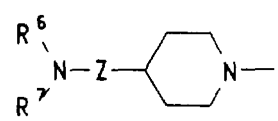
(IV)

bedeutet, worin

| | |
|---|---|
| $R^6$ | für Wasserstoff oder tert. Butyloxycarbonyl steht, |
| $R^7$ | Wasserstoff bedeutet, |
| $R^8$ | Wasserstoff oder Benzyl bedeutet, |
| $R^9$ | Wasserstoff bedeutet, |
| $R^{10}$ und $R^{11}$ | zusammen Wasserstoff oder Methyl bedeuten, und |
| Z | $-CH_2-$ ist, |
| X | -CO- oder $-SO_2-$ ist, |
| Y | $-CH_2-$ oder -O- bedeutet, |
| $R^2$ | Benzyl oder 1- oder 2-Naphthylmethyl bedeutet, |
| $R^3$ | Cyclohexylmethyl bedeutet, |
| $R^4$ | Hydroxy ist, |
| $R^5$ | für einen Rest der Formel V |

$$(CH_2)_s - CHR^{15} - Het \qquad (V)$$

steht, worin

| | |
|---|---|
| $R^{15}$ | Wasserstoff bedeutet, |
| Het | für einen 2-Pyridyl-Rest steht, und |
| s | 1 bedeutet, und |

B einen Rest einer Aminosäure H-B-OH aus der Reihe Histidin, Norvalin, oder S-Methylcystein bedeutet, oder deren physiologisch verträgliche Salze.

Die Chiralitätszentren in den Verbindungen der Formel I können die R-, S- oder R,S-Konfiguration aufweisen. B liegt vorzugsweise in der S-Konfiguration bzw., im Falle von Cystein und davon abgeleiteten Aminosäuren, in der R-Konfiguration vor.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in welcher $R^{10}$ und $R^{11}$ Wasserstoff bedeuten und X für -CO- steht.

Besonders bevorzugt sind Verbindungen der Formel I aus der Reihe N-[N-(3-(4-(tert.-Butyloxycarbonyl)amino-1-piperidinyl-carbonyl)-2(R)-(1-tetrahydronaphthylmethyl)propionyl)-L-histidinyl]-(2S,3R,4S)-1-cyclohexyl-3,4-dihydroxy-6-(2-pyridyl)-2-hexylamid und N-[N-(3-(4-Amino-1-piperidinyl-carbonyl)-2(R)-(1 -tetrahydronaphthylmethyl)propionyl)-L-histidinyl]-(2S,3R,4S)-cyclohexyl-3,4-dihydroxy-6-(2-pyridyl)-2-hexylamid-acetat und ganz besonders bevorzugt ist N-[N-(3-(4-Amino-1-piperidinyl-carbonyl)-2(R)-benzylpropionyl)-L-histidinyl]-(2S,3R,4S)-cyclohexyl-3,4-dihydroxy-6-(2-pyridyl)-2-hexylamid-acetat.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehende Formel IVa bzw. IVb,

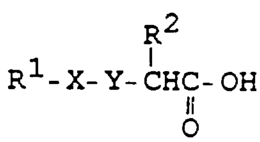

(IVa)

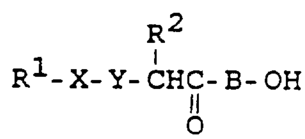

(IVb)

Fragmente einer Verbindung der Formel I mit einer endständigen Aminogruppe besitzen die nachstehende Formel Va bzw. Vb:

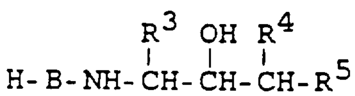

Va

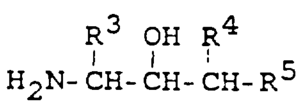

Vb

Die Verknüpfung der Komponenten erfolgt dabei nur in der Kombination IVa mit Va bzw. IVb mit Vb. Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:

Aktivestermethode mit N-Hydroxy-succinimid oder 1-Hydroxybenzotriazol als Esterkomponente, Kupplung mit einem Carbodiimid wie Dicyclohexylcarbodiimid oder mit Propanphosphonsäureanhydrid oder Methylethylphosphinsäureanhydrid und die Gemischt-Anhydrid-Methode mit Pivaloylchlorid (M. Zaoral, Coll. Chem. Commun., 1962, 27, 1273). Man führt die Reaktion in einem inerten Lösungsmittel oder Lösungsmittelgemisch bei einer Temperatur zwischen -20 °C und dem Siedepunkt des Reaktionsgemisches durch.

Die Herstellung der als Ausgangsverbindungen verwendeten optisch aktiven Amine der Formel Vb,
worin $R^2$, $R^3$ und $R^4$ wie oben definiert sind, erfolgt ausgehend von optisch aktiven α-Aminosäuren, wobei deren Asymmetriezentrum erhalten bleibt. Hierzu wird in bekannter Weise ein N-geschützter Aminoaldehyd hergestellt, welcher in einer Aldol-analogen Addition an einen entsprechenden Heteroarylalkyl-Baustein gekuppelt wird und nach Abspaltung der N-Schutzgruppe Aminoalkohole der Formel Vb ergibt. Bei $R^4$ = OH dient ebenfalls ein N-geschützter Aminoaldehyd als Ausgangsmaterial, der z.B. durch aldol-analoge Addition von ungesättigten Verbindungen, Einführung geeigneter Schutzgruppen und anschließende Epoxidierung in die benötigten Zwischenprodukte umgewandelt wird. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et. al., J. Org. Chem. 34, 2543 (1969)).

Die Herstellung N-geschützter Aminoaldehyde erfolgt nach B. Castro et al. (Synthesis 1983, 676).

Die Aldol-analoge Addition an N-geschützte Aminoaldehyde (bevorzugterweise N-tert.-Butoxycarbonylund Benzyloxycarbonyl-Schutzgruppen) erfolgt in einem gegenüber Basen inerten Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-Otert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die Herstellung Carboxy-gechützter Bernsteinsäurederivate in enantiomerenreiner Form erfolgt nach J.J. Plattner et al. (J. Med. Chem 31, 2277 (1988)) und D.A. Evans et al. (J. Am. Chem. Soc. 104, 1737 (1982)).

Die Herstellung carbamoylsubstituierter $\alpha$-Hydroxypropionsäurederivate erfolgt durch Umsetzung von enantiomerenreinen oder racemischen $\alpha$-Hydroxypropionsäurederivaten und den wie definiert substituierten N-haltigen Ringsystemen mit reaktiven Kohlensäurederivaten wie z.B. Phosgen, Diphosgen, Triphosgen, Carbonyldiimidazol oder Di-(1-benzotriazolyl)carbonat unter Zusatz geeigneter Basen wie z.B. Triethylamin, Pyridin oder Ethyl-diisopropylamin in einem inerten Lösungsmittel.

Aminosulfonylderivate werden durch Kondensation der entsprechend substituierten Amine mit $\omega$-Chlorsulfonylalkylcarbonsäurederivaten unter Verwendung von Basen hergestellt und anschließend nach gegebener Abspaltung von temporär eingeführten Schutzgruppen nach dem oben beschriebenen Verfahren mit Fragmenten der allgemeinen Formel I mit endständiger Aminogruppe (wie z.B. Vb) unter Verwendung von geeigneten Aktivierungsmethoden gekuppelt. U.a. werden die Chlorsulfonylpropionsäurederivate z.B. durch Michael-Addition geeigneter schwefelhaltiger Nukleophile an substituierte Acrylsäure-derivate mit anschließender Oxidation am S-Atom gewonnen.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt.

Stereoisomerengemische, insbesondere Diastereomerengemische, die bei Verwendung racemischer Carbonsäurederivate und racemischer Aminosäuren H-B-OH anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der AspartylProteasen, welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37°C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung

wird durch Renin-Inhibitoren gehemmt.

**2. Gewinnung des Plasmas**

Frisch zu entnehmendes Humanblut (ca. 0,5 l pro Person; Bluko-Entnahmegerät der Fa. ASID Bonz und Sohn, Unterschleißheim) wird in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4°C, 15 min; wiederholen, falls erforderlich) wird das Plasma vorsichtig abpipettiert und in geeigneten Portionen bei -30°C eingefroren. Für den Test werden nur Plasmen mit ausreichend hoher Reninaktivität verwendet. Plasmen mit niedriger Reninaktivität werden durch eine Kältebehandlung (-4°C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:
Inkubationsansatz:

1000 µl Plasma (bei 0-4°C aufgetaut)
100 µl Phosphatpuffer (pH 7,4) Zusatz von $10^{-4}$ M Ramiprilat
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37°C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25°C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische- Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel I können als Antihypertensiva, sowie zur Behandlung der Herzinsuffizienz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym, deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirkung, die die erfindungsgemäßen Verbindungen insbesondere zur Therapie und Prophylaxe von durch Infektion mit

HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-9}$ Mol/l.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucoseoder Mannitlösungen, oder auch eine Mischung aus den verschiedenen genannten Lösungsmitteln.

**Verzeichnis der verwendeten Abkürzungen:**

| | |
|---|---|
| Boc | tert.-Butyloxycarbonyl |
| BuLi | n-Butyl-Lithium |
| DCC | Dicyclohexylcarbodiimid |
| DCI | Desorption Chemical Ionisation |
| DNP | 2,4-Dinitrophenyl |
| DME | Dimethoxyethan |
| DMF | Dimethylformamid |
| EE | Essigsäureethylester |
| FAB | Fast atom bombardment |
| F | moc Fluorenylmethoxycarbonyl |
| h | Stunde |
| HOBt | 1-Hydroxybenzotriazol |
| M | Molekularpeak |
| MS | Massenspektrum |
| min | Minuten |
| NEM | N-Ethylmorpholin |
| THF | Tetrahydrofuran |
| Trt | Triphenylmethyl |

Die folgenden Beispiele sollen die Herstellung der erfindungsgemäßen Verbindungen illustrieren, ohne daß die Erfindung auf diese beschränkt wäre.

**Beispiel 1**

N-[N-(2(R)-Benzyl-3-(4-(tert.-butyloxycarbonyl)amino-1-piperidinyl-carbonyl)-propionyl)-L-histidinyl]-(2S,3R,4S) -1-cyclohexyl-3,4-dihydroxy-6-(2-pyridyl)-2-hexylamid

0,12 g der Verbindung aus Beispiel 1a werden mit 60 mg Thiophenol in 4 ml abs. Acetonitril 2 Stunden bei Raumtemperatur gerührt. Nach Einengen und zweimaligem Codestillieren mit Toluol wird an Kieselgel mit $CH_2Cl_2$/MeOH/ges. $NH_3$ (10:1:0,1) chromatographiert. Man erhält 72 mg der Titelverbindung als gelben amorphen Feststoff.

$R_f$ ($SiO_2$, $CH_2Cl_2$/MeOH/ges. $NH_3$ 10:1:0,1): 0,33
MS (FAB): 802 (M+H)

a) N-[N-(2(R)-Benzyl-3-(4-(tert.-butyloxycarbonyl)amino-1-piperidinyl-carbonyl)-propionyl)-L-histidinyl(DNP)]-(2S,3R,4S)-1-cyclohexyl-3,4-dihydroxy-6-(2-pyridyl)-2-hexylamid

0,4 g BOC-L-His(DNP)-(2S,3R,4S)-1-cyclohexyl-3,4-dihydroxy-6(2-pyridyl)-2-hexylamid (Verbindung aus Beispiel 1f) werden mit 6 ml Trifluoressigsäure in 6 ml abs. $CH_2Cl_2$ für 2 Stunden bei Raumtemperatur gerührt. Der nach Einengung resultierende Rückstand wird in 1 m $NaHCO_3$-Lösung aufgenommen, das Gemisch dreimal mit EE extrahiert und die EE-Phase über $Na_2SO_4$ getrocknet. Nach Einengung wird der Rückstand in 6 ml abs. DMF gelöst, 224,5 mg der Verbindung aus Beispiel 1b, 118,6 mg N,N′-Dicyclohexylcarbodiimid und 89,3 mg 1-Hydroxybenzotriazol hinzugegeben, der pH der Lösung mit N-Ethylmorpholin auf 9 eingestellt und für 48 Stunden stehengelassen. Nach Filtration wird mit EE verdünnt und je 1 mal mit gesättigter $NaHCO_3$-Lösung, Wasser und gesättigter Kochsalz-Lösung gewaschen, über $Na_2SO_4$ getrocknet und eingeengt. Chromatographie an Kieselgel ($CH_2Cl_2$/MeOH 20:1) ergibt 140 mg der Titelverbindung als gelbes Harz.
$R_f$ ($SiO_2$, $CH_2Cl_2$/MeOH 10:1): 0,42
MS (FAB): 968 (M+H), 974 (M+Li)

b) 2 (R)-Benzyl-3-[4-(tert.-butyloxycarbonyl) amino-1-piperidinyl-carbonyl]-propionsäure

1,3 g der Verbindung aus Beispiel 1c werden in 60 ml abs. Ethanol gelöst und mit 200 mg Pd/C (10 % Pd) 1 Stunde bei Raumtemperatur hydriert (1,1 bar $H_2$). Nach Filtrieren und Abzug des Lösungsmittels im Vakuum kristallisieren 0,74 g der Titelverbindung aus kaltem Diethylether aus.
$R_f$ ($SiO_2$, $CH_2Cl_2$/MeOH 9:1): 0,3
Fp.: 135-136 °C
MS (DCI): 391 (M+H)

c) 2(R)-Benzyl-3-[4-(tert.-butyloxycarbonyl)amino-1-piperidinyl-carbonyl]-propionsäurebenzylester

1,0 g (2R)-2-(Carboxymethyl)-3-phenyl-propionsäurebenzylester (hergestellt nach J. Med. Chem. 31 (1988) 2277) werden in 50 ml abs. $CH_2Cl_2$ bei 0 °C mit 0,31 ml Oxalylchlorid und 0,5 ml abs. DMF 1 Stunde gerührt. Nach Einengen wird der Rückstand in 25 ml abs. $CH_2Cl_2$ aufgenommen, die Lösung mit Triethylamin auf pH 7 eingestellt und 0,71 g der Verbindung aus Beispiel 1d und 0,47 ml Triethylamin, gelöst in 50 ml abs. $CH_2Cl_2$, hinzugetropft. Nach 3 Stunden Rühren bei 0 °C wird der Ansatz eingeengt, der Rückstand in EE aufgenommen, jeweils einmal mit kalter 2 N HCl- und gesättigter $NaHCO_3$-Lösung gewaschen und über $MgSO_4$ getrocknet. Abzug des Lösungsmittels ergab 1,35 g der Titelverbindung als schwach gelbes Öl.
$R_f$ ($SiO_2$, $CH_2Cl_2$/MeOH 9:1): 0,5
MS (DCI): 481 (M+H)

d) 4-(tert.-Butyloxycarbonyl)amimo-piperidin

10,0 g der Verbindung aus Beispiel le werden in 60 ml Ethanol/Eisessig 9:1 mit 1,0 g Pd/C (10 % Pd) 1 Stunde bei Raumtemperatur hydriert (1,1 bar $H_2$). Nach Filtration, Einengen, 2-maligem Codestillieren mit Toluol, Aufnahme in EE, Ausschütteln mit ges. $NaHCO_3$- und gesättigter Kochsalzlösung, Trocknung über $MgSO_4$ und Einengung resultiert ein beiger Rückstand, aus dem 5,5 g der Titelverbindung in Form weißer Kristalle durch Umkristallisation aus EE gewonnen werden.
$R_f$ ($SiO_2$, $CH_2Cl_2$ /MeOH 9:1): 0,11
Fp.: 159-161 °C
MS (DCI): 201 (M+H)

e) 1-Benzyl-4-[(tert.-butyloxycarbonyl)amino]-piperidin

10,0 g 4-Amino-N-benzylpiperidin werden in 100 ml abs. $CH_2Cl_2$ gelöst, mit 11,5 g Di-tert.-butyldicarbonat versetzt, die Lösung 2 Stunden bei Raumtemperatur gerührt und 12 Stunden stehengelassen. Einengung und Umkristallisation des Rückstandes aus EE liefert 12,9 g der Titelverbindung als weiße Kristalle.
$R_f$ ($SiO_2$, $CH_2Cl_2$/MeOH 8:2): 0,23
Fp.: 123 °C
MS (DCI): 291 (M+H)

f) BOC-L-His(DNP)-(2S,3R,4S)-1-cyclohexyl-3,4-dihydroxy-6-(2-pyridyl)-2-hexylamid

0,5 mmol des (2S,3R,4S)-Isomeren aus Beispiel 1g werden mit 5 ml HCl in DME (gesättigt) 2 Stunden gerührt. Nach Einengen wird der Rückstand in 3 ml abs. DMF gelöst. Je 0,5 mmol BOC-His(DNP)-OH, N,N'-Dicyclohexylcarbodiimid und 1-Hydroxybenzotriazol werden zugegeben. Die Lösung wird mit N-Ethylmorpholin auf pH 9 gestellt und 24 Stunden gerührt. Nach Filtration wird mit EE verdünnt, je 1 Mal mit gesättigter $NaHCO_3$-Lösung, Wasser und gesättigter Kochsalzlösung gewaschen, über $MgSO_4$ getrocknet und eingeengt. Chromatographie an Kieselgel ($CH_2Cl_2$/MeOH 20:1) liefert die Titelverbindung als gelbes Harz.
MS (FAB): 696 (M+H)

g) (2S,3R,4S)-2-(tert.-Butyloxycarbonyl)amino-1-cyclohexyl-3,4-dihydroxy-6(2-pyridyl)-hexan

93,0 mg 2-Picolin in 10 ml THF werden bei -78 °C mit 1,4 ml n-Butyllithium versetzt. Nach Aufwärmen auf Raumtemperatur wird 30 Minuten gerührt, dann auf -40 °C abgekühlt. 1 mmol (2RS,3R,4S)-3-tert.-Butyldimethyl-silyloxy-4-(tert.-butyloxycarbonylamino)-5-cyclohexyl-1,2-oxopentan (bekannt aus EP-A 189 203, Beispiel 6) werden zugegeben (gelöst in 5 ml THF). Nach 10 Stunden Rühren bei Raumtemperatur wird mit Wasser verdünnt und mit Methyl-tert.-butylether extrahiert. Das Rohprodukt wird nach Einengen in THF gelöst und mit 5 ml einer 1 M Lösung von Tetrabutylammoniumfluorid in THF 1 Stunde bei 0 °C gerührt. Nach Verdünnen mit Wasser, Extrahieren mit EE und Einengen erhält man 0,15 g des (2S,3R,4S)-Isomeren
(MS (FAB): 391 (M+H)) und 0,12 g des (2S,3S,4S)-Isomeren
(MS (FAB): 391 (M+H)).

**Beispiel 2**

N-[N-(3-(4-Amino-1-piperidinyl-carbonyl)-2(R)-benzylpropionyl)-L-histidinyl]-(2S,3R,4S)-1-cyclohexyl-3,4-dihydroxy-6 (2-pyridyl)-2-hexylamid-acetat

70,0 mg der Verbindung aus Beispiel 1 werden in 4 ml abs. $CH_2Cl_2$ mit 4 ml Trifluoressigsäure 2 Stunden bei Raumtemperatur gerührt. Nach Einengen wird 2 Mal in Toluol aufgenommen und jeweils wieder eingeengt. Der Rückstand wird in wenig Wasser gelöst, der pH der Lösung durch Zusatz von Amberlite-Ionenaustauscher (Acetat-Form) auf 5 eingestellt, der Ionenaustauscher abfiltriert und das Filtrat gefriergetrocknet. Es resultieren 63,8 mg der Titelverbindung in Form eines blaßgelben, amorphen Feststoffes.
MS (FAB): 702 (M+H)

**Beispiel 3**

N-[N-(2(S)-(4-(tert.-Butyloxycarbonyl)amino-1-piperidinyl-carbonyl-oxy)-3-phenyl-propionyl)-L-histidinyl]-(2S,3R,4S) -1-cyclohexyl-3,4-dihydroxy-6-(2-pyridyl)-2-hexylamid

Diese Verbindung wird aus der Verbindung aus Beispiel 3a nach dem in Beispiel 1 angegebenen Verfahren hergestellt; gelber amorpher Feststoff.
MS (FAB): 804 (M+H)

a) N-[N-(2(S)-(4-(tert.-Butyloxycarbonyl)amino-1-piperidinyl-carbonyl-oxy)-3-phenyl-propionyl)-L-histidinyl(DNP)]-(2S,3R,4S)-1-cyclohexyl-3,4-dihydroxy-6(2-pyridyl)-2-hexylamid

Diese Verbindung wird nach dem in Beispiel la angeführten Verfahren aus den Verbindungen aus den Beispielen 1f und 3b hergestellt; gelbes Harz.
$R_f$ ($SiO_2$, $CH_2Cl_2$/MeOH 10:1): 0,35
MS (FAB): 970 (M+H);

b) 2(S)-[4-(tert.-Butyloxycarbonyl)amino-1-piperidinyl-carbonyl-oxy]-3-phenyl-propionsäure

465,0 mg der Verbindung aus Beispiel 3c werden in 5 ml Methanol bei 0 °C mit 5 ml 1 N Natronlauge 3 Stunden gerührt. Nach Einengen und Aufnahme des Rückstandes in Wasser wird die Lösung mit 2 N Salzsäure auf pH 5 eingestellt und mehrmals mit EE extrahiert. Die organische Phase wird mit gesättigter Kochsalzlösung gewaschen, über $MgSO_4$ getrocknet und eingeengt. Umkristallisieren des Rückstandes aus EE/n-Heptan ergibt 242,0 mg der Titelverbindung.

$R_f$ ($SiO_2$, $CH_2Cl_2$/MeOH 9:1): 0,29
Fp.: 100-103 °C
MS (DCI): 293 (M-BOC+2H), 201 (M-$C_{10}H_9O_4$+2H)

c) 2(S)-[4-(tert.-Butyloxycarbonyl)amino-1-piperidinyl-carbonyl-oxy]-3-phenyl-propionsäureethylester

500,0 mg 2(S)-Hydroxy-3-phenyl-propionsäureethylester (hergestellt aus L-3-Phenylmilchsäure und ethanolischer HCl-Lösung) werden in 10 ml abs. $CH_2Cl_2$ mit 1,1 g Di-(1-benzotriazolyl)-carbonat (70 %ig) und 332,7 mg Ethyldiisopropylamin versetzt. Die resultierende Lösung wird zunächst 7 Stunden bei Raumtemperatur gerührt und dann 12 Stunden stehengelassen. Nach Zugabe von 512,5 mg der Verbindung aus Beispiel ld und 332,7 mg Ethyldiisopropylamin wird weitere 7 Stunden bei Raumtemperatur gerührt. Die Reaktionslösung wird mit 30 ml EE verdünnt, mit gesättigter $Na_2CO_3$- und gesättigter Kochsalzlösung gewaschen und über $Na_2SO_4$ getrocknet. Nach Einengen und Chromatographie an Kieselgel (n-Heptan/EE 2:1) resultiert 480,0 mg der Titelverbindung als farbloses Öl.
$R_f$ ($SiO_2$, n-Heptan/EE 2:1): 0,20
MS (DCI): 421 (M+H), 321 (M-BOC+H)

**Beispiel 4**

N-[N-(2(S)-(4-Amino-1-piperidinyl-carbonyl-oxy)-3-phenyl-propionyl)-L-histidinyl]-(2S,3R,4S)-1-cyclohexyl-3,4-dihydroxy-6-(2-pyridyl)-2-hexylamid-acetat

Diese Verbindung wird nach dem in Beispiel 2 ängeführten Verfahren aus der Verbindung aus Beispiel 3 dargestellt; beiger, amorpher Feststoff.
MS (FAB): 704 (M+H)

**Beispiel 5**

N-[N-(2(R)-Benzyl-3-(N-benzyl-piperidinyl-4-amino-carbonyl)propionyl)-L-histidinyl]-(2S,3R,4S)-1-cyclohexyl-3,4-dihydroxy-6-(2-pyridyl)-2-hexylamid

Diese Verbindung wird nach dem in Beispiel 1 angegebenen Verfahren aus der Verbindung aus Beispiel 5a hergestellt; gelbes Harz.
MS (FAB): 792 (M+H)

a) N-[N-(2(R)-Benzyl-3-(N-benzyl-piperidinyl-4-amino-carbonyl)propionyl)-L-histidinyl(DNP)]-(2S,3R,4S)-1-cyclohexyl-3,4-dihydroxy-6-(2-pyridyl)-2-hexylamid

Diese Verbindung wird nach dem in Beispiel 1a angegebenen Verfahren aus den Verbindungen aus den Beispielen 1g und 5b hergestellt.
MS (FAB): 958 (M+H), 964 (M+Li)

b) 2(R)-Benzyl-3(N-benzyl-piperidinyl-4-amino-carbonyl)propionsäure

345,0 mg der Verbindung aus Beispiel 5c werden in 30 ml Ethanol mit 70 mg Pd/C (10 % Pd) 10 Minuten bei Raumtemperatur hydriert (1,1 bar $H_2$). Filtration und Einengung ergibt 269,0 mg der Titelverbindung in Form eines hellbraunen Öls.
$R_f$ ($SiO_2$, $CH_2Cl_2$/MeOH 1:1): 0,21
MS (DCI): 381 (M+H)

c) 2(R)-Benzyl-3-(N-benzyl-piperidinyl-4-amino-carbonyl) propionsäurebenzylester

Diese Verbindung wird aus 4-Amino-N-benzylpiperidin nach dem in Beispiel 1c beschriebenen Verfahren hergestellt, das hier durch die Verwendung von 2 Äquivalenten Base, den Verzicht auf die Extraktion mit 2 N HCl-Lösung sowie die chromatographischen Aufarbeitung an Kieselgel ($CH_2Cl_2$/EE 7:3) modifiziert wird.
$R_f$ ($SiO_2$, $CH_2Cl_2$/EE 7:3): 0,25
MS (DCI): 471 (M+H)

**Beispiel 6**

N-[N-(2(R)-Benzyl-3-(piperidinyl-4-amino-carbonyl)propionyl)-L-histidinyl]-(2S,3R,4S)-1-cyclohexyl-3,4-dihydroxy-6-(2-pyridyl)-2-hexylamid-acetat

40,0 mg der Verbindung aus Beispiel 5 werden in 10 ml Ethanol/Eisessig 9:1 mit 10 mg Pd/C (10 % Pd) 1 Stunde bei Raumtemperatur hydriert (1,1 bar $H_2$). Nach Filtration, Einengen und 2-maligem Codestillieren mit Toluol wird der Rückstand in Wasser aufgenommen und die Lösung gefriergetrocknet. Es resultieren 22,3 mg der Titelverbindung als beiger, amorpher Feststoff.
MS (FAB): 702 (M+H)

**Beispiel 7**

N-[N-(2(S)-(N-Benzyl-piperidinyl-4-amino-carbonyl-oxy)-3-phenyl-propionyl)-L-histidinyl]-(2S,3R,4S)-1-cyclohexyl-3,4-dihydroxy-6-(2-pyridyl)-2-hexylamid

Diese Verbindung wird aus der Verbindung aus Beispiel 7a nach dem im Beispiel 1 angegebenen Verfahren hergestellt; gelber, amorpher Feststoff
MS (FAB): 794 (M+H)

a) N-[N-(2(S)-N-Benzyl-piperidinyl-4-amino-carbonyl-oxy)-3-phenyl-propionyl)-L-histidinyl(DNP)]-(2S,3R,4S)-1-cyclohexyl-3,4-dihydroxy-6-(2-pyridyl)-2-hexylamid

Diese Verbindung resultiert aus den Verbindungen aus den Beispielen 1g und 7b nach dem in Beispiel 1a angegebenen Verfahren; gelbes Harz.
MS (FAB): 960 (M+H); 966 (M+Li)

b) 2(S)-(N-Benzyl-piperidinyl-4-amino-carbonyl-oxy)-3-phenyl-propionsäure

400,0 mg der Verbindung aus Beispiel 7c werden in 5 ml Ethanol mit 1 ml 1N Natronlauge bei 0 °C 3 Stunden gerührt und für 12 Stunden bei ca. 8 °C stehengelassen. Nach Einengung wird der Rückstand in Wasser gelöst, mit 2 N Salzsäure neutralisiert und nach Kochsalzzusatz mehrmals mit EE extrahiert. Trocknung über $Na_2SO_4$, Einengung und Chromatographie an Kieselgel ($CH_2Cl_2$/MeOH 2:1) liefert 224,0 mg der Titelverbindung.
MS (DCI): 383 (M+H)

c) 2(S)-(N-Benzyl-piperidinyl-4-amino-carbonyl-oxy)-3-phenyl-propionsäureethylester

Diese Verbindung wird aus 4-Amino-N-benzylpiperidin nach dem in Beispiel 3c angeführten Verfahren hergestellt, wobei hier Pyridin als Hilfsbase und bei der chromatographischen Aufarbeitung das Laufmittel n-Heptan/EE 1:1 verwendet werden; weiße Kristalle.
$R_f$ ($SiO_2$, n-Heptan/EE 1:1) : 0,25
Fp.: 72-74 °C
MS (DCI): 411 (M+H)

**Beispiel 8**

N-[N-(3-Phenyl-2(S)-(piperidinyl-4-amino-carbonyl-oxy)-propionyl)-L-histidinyl]-(2S,3R,4S)-1-cyclohexyl-3,4-dihydroxy-6-(2-pyridyl)-2-hexylamid-acetat

Diese Verbindung wird aus der Verbindung aus Beispiel 7 nach dem in Beispiel 6 angeführten Verfahren dargestellt; hellbrauner, amorpher Feststoff.
MS (FAB): 702 (M+H)

**Referenzbeispiel 9**

N-[N-(2(R)-Benzyl-3-(4-(tert.-butyloxycarbonyl)amino-1-piperidinyl-carbonyl)-propionyl)-L-histidinyl]-(2S,3S)-1-cyclohexyl-3-hydroxy-6-(2-pyridyl)-2-hexylamid

80 mg der Verbindung aus Beispiel 9a werden in 1 ml 90 %iger Essigsäure 3 Stunden bei 60 °C gerührt. Nach Einengen wird der Rückstand in EE aufgenommen, je zweimal mit gesättigter $NaHCO_3$- und Kochsalz-Lösung gewaschen und über $Na_2SO_4$ getrocknet. Einengung und Chromatographie an Kieselgel ($CH_2Cl_2$/MeOH 9:1) liefert 31,2 mg der Titelverbindung.
MS (FAB): 786 (M+H)

a) N-[N-(2(R)-Benzyl-3-(4-(tert.-butyloxycarbonyl)-amino-1-piperidinyl-carbonyl)-propionyl)-L-histidinyl(Trt)]-(2S,3S)-1-cyclohexyl-3-hydroxy-6-(2-pyridyl)-2-hexylamid

375 mg der Verbindung aus Beispiel 9b werden in 4 ml abs. DMF gelöst, 225 mg der Verbindung aus Beispiel 1b, 120 mg N,N'-Dicyclohexylcarbodiimid und 90 mg 1-Hydroxybenzotriazol hinzugegeben und mit N-Ethylmorpholin die Lösung auf pH 9 eingestellt. Nach 48 Stunden Stehenlassen bei Raumtemperatur wird filtriert, das Filtrat mit EE verdünnt und je 1 Mal mit gesättigter $NaHCO_3$-Lösung, Wasser und gesättigter Kochsalzlösung gewaschen. Trocknung, Einengen und Chromatographie an Kieselgel ergibt 298 mg der Titelverbindung.
MS (FAB): 1028 (M+H)

b) H-His(Trt)-(2S,3S)-1-cyclohexyl-3-hydroxy-6-(2-pyridyl)-2-hexylamid

600 mg der in Beispiel 9c beschriebenen Verbindung werden in 5 ml abs. DMF mit 0,65 ml Diethylamin 1 Stunde bei Raumtemperatur gerührt. Einengen und Chromatographie an Kieselgel ($CH_2Cl_2$/MeOH 9:1) liefert 380 mg der Titelverbindung.
MS (FAB): 656 (M+H)

c) Fmoc-His(Trt)-(2S,3S)-1-cyclohexyl-3-hydroxy-6-(2-pyridyl)-2-hexylamid

1,0 g Fmoc-His(Trt)-OH, 283 mg 1-Hydroxybenzotriazol, 370 mg N,N'-Dicyclohexylcarbodiimid und 0,25 ml N-Ethylmorpholin werden in 10 ml abs. DMF gelöst und 1 Stunde bei Raumtemperatur gerührt. Zu dieser Mischung wird eine Lösung von 483 mg 2(S)-Amino-1-cyclohexyl-3(S)-hydroxy-6(2-pyridyl)-hexan (bekannt aus EP-A 0 255 082) in 4 ml DMF getropft und 12 Stunden bei Raumtemperatur gerührt. Nach Zugabe von 5 ml Wasser wird vom gebildeten Harnstoff abfiltriert und mit EE aufgenommen. Die organische Phase wird wiederholt mit gesättigter $NaHCO_3$- und gesättigter Kochsalzlösung gewaschen, über $MgSO_4$ getrocknet und nach Filtration im Vakuum eingeengt. Chromatographie an Kieselgel liefert 880 mg der genannten Verbindung.
Schmelzpunkt: 85 °C
MS (FAB):878

**Referenzbeispiel 10**

N-[N-(3-(4-Amino-1-piperidinyl-carbonyl)-2(R)-benzyl-propionyl)-L-histidinyl]-(2S,3S)-1-cyclohexyl-3-hydroxy-6-(2-pyridyl)-2-hexylamid-acetat

25 mg der Verbindung aus Beispiel 9 werden in 2 ml abs. $CH_2Cl_2$ mit 2 ml Trifluoressigsäure 2 Stunden bei Raumtemperatur gerührt. Nach Einengen, 2-maligem Codestillieren mit Toluol wird der Rückstand in Wasser gelöst, durch Zugabe von Amberlite-Ionenaustauscher (Acetat-Form) die Lösung auf pH 5 eingestellt, filtriert und das Filtrat lyophilisiert. Die Titelverbindung erhält man in einer Ausbeute von 20 mg.
MS (FAB): 686 (M+H)

**Referenzbeispiel 11**

N-[N-(2(S)-(4-(tert.-Butyloxycarbonyl)amino-1-piperidinyl-carbonyl-oxy)-3-phenyl-propionyl)-L-histidinyl]-(2S,3S)-1-cyclohexyl-3-hydroxy-6-(2-pyridyl)-2-hexylamid

Diese Verbindung wird aus der Verbindung aus Beispiel 11a nach dem in Beispiel 9 angegebenen Verfahren hergestellt.

MS (FAB): 788 (M+H)

a) N-[N-(2(S)-(4-(tert.-Butyloxycarbonyl)amino-1-piperidinyl-carbonyl-oxy)-3-phenyl-propionyl)-L-histidinyl(Trt)]-(2S,3S)-1-cyclohexyl-3-hydroxy-6-(2-pyridyl)-2-hexylamid

Diese Verbindung wird aus den Verbindungen aus den Beispielen 3b und 9b nach dem im Beispiel 9a angegebenen Verfahren dargestellt.
MS (FAB): 1030 (M+H)

**Referenzbeispiel 12**

N-[N-(2(S)-(4-Amino-1-piperidinyl-carbonyl-oxy)-3-phenyl-propionyl)-L-histidinyl]-(2S,3S)-1-cyclohexyl-3-hydroxy-6-(2-pyridyl)-2-hexylamid-acetat

Die Titelverbindung wird nach dem in Beispiel 2 beschriebenen Verfahren aus der im Beispiel 11 beschriebenen Verbindung hergestellt.
MS (FAB): 688 (M+H)

**Referenzbeispiel 13**

N-[N-(2(R)-Benzyl-3-(N-benzyl-piperidinyl-4-amino-carbonyl)-propionyl)-L-histidinyl]-(2S,3S)-1-cyclohexyl-3-hydroxy-6-(2-pyridyl)-2-hexylamid

Die Titelverbindung wird nach dem im Beispiel 9 beschriebenen Verfahren aus der im Beispiel 13a angeführten Verbindung hergestellt.
MS (FAB): 776 (M+H)

a) N-[N-(2(R)-Benzyl-3-(N-benzyl-piperidinyl-4-amino-carbonyl)propionyl)-L-histidinyl(Trt)]-(2S,3S)-1-cyclohexyl-3-hydroxy-6-(2-pyridyl)-2-hexylamid

Diese Verbindung wird aus den Verbindungen aus den Beispielen 5b und 9b nach dem in Beispiel 9a beschriebenen Verfahren hergestellt.
MS (FAB): 1018 (M+H)

**Referenzbeispiel 14**

N-[N-(2(R)-Benzyl-3-(piperidinyl-4-amino-carbonyl)-propionyl)-L-histidinyl]-(2S,3S)-1-cyclohexyl-3-hydroxy-6-(2-pyridyl)-2-hexylamid-acetat

Die Titelverbindung wird aus der Verbindung aus-Beispiel 13 nach dem im Beispiel 6 angegebenen Verfahren hergestellt.
MS (FAB): 686 (M+H)

**Referenzbeispiel 15**

N-[N-(2(S)-(N-Benzyl-piperidinyl-4-amino-carbonyl-oxy)-3-phenyl-propionyl)-L-histidinyl]-(2S,3S)-1-cyclohexyl-3-hydroxy-6-(2-pyridyl)-2-hexylamid

Die Titelverbindung wird aus der im Beispiel 15a angegebenen Verbindung nach dem im Beispiel 9 beschriebenen Verfahren hergestellt.
MS (FAB): 778 (M+H)

a) N-[N-(2(S)-(N-Benzyl-piperidinyl-4-amino-carbonyl-oxy)-3-phenyl-propionyl)-L-histidinyl(Trt)]-(2S,3S)-1-cyclohexyl-3-hydroxy-6-(2-pyridyl)-2-hexylamid

Die Titelverbindung wird aus den in den Beispielen 7b und 9b angeführten Verbindungen nach dem im Beispiel 9a beschriebenen Verfahren hergestellt.
MS (FAB): 1020 (M+H)

**Referenzbeispiel 16**

N-[N-(3-Phenyl-2(S)-(piperidinyl-4-amino-carbonyl-oxy)-propionyl)-L-histidinyl]-(2S,3S)-1-cyclohexyl-3-hydroxy-6-(2-pyridyl)-2-hexylamid-acetat

Die Titelverbindung wird aus der Verbindung aus Beispiel 15 nach dem in Beispiel 6 beschriebenen Verfahren hergestellt. MS (FAB): 688 (M+H)

**Beispiel 17**

N-[N-(2(R)-Benzyl-3-(4-(tert.-butyloxycarbonyl)amino-1-piperidinyl-carbonyl)-propionyl)-L-norvalinyl]-(2S,3R,4S)-1-cyclohexyl-3,4-dihydroxy-6-(2-pyridyl)-2-hexylamid

Die Titelverbindung wird aus den Verbindungen aus den Beispielen 1b und 17a nach dem in Beispiel 1a angegebenen Verfahren hergestellt.

a) BOC-Nva-(2S,3R,4S)-1-cyclohexyl-3,4-dihydroxy-6(2-pyridyl)-2-hexylamid

Die Titelverbindung wird aus BOC-Norvalin und der Verbindung aus Beispiel 1g nach dem in Beispiel 1a angegebenen Verfahren hergestellt.
MS (FAB): 492 (M+H)

**Beispiel 18**

N-[N-(3-(4-Amino-1-piperidinyl-carbonyl)-2(R)-benzyl-propionyl)-L-norvalinyl]-(2S,3R,4S)-1-cyclohexyl-3,4-dihydroxy-6-(2-pyridyl)-2-hexylamid-acetat

Die Titelverbindung wird aus der Verbindung aus Beispiel 17 nach dem in Beispiel 2 angegebenen Verfahren hergestellt.
MS (FAB): 664 (M+H)

**Beispiel 19**

N-[N-(2(S)-(4-(tert.-Butyloxycarbonyl)amino-1-piperidinyl-carbonyl-oxy)-3-phenyl-propionyl)-L-norvalinyl]-(2S,3R,4S)-1-cyclohexyl-3,4-dihydroxy-6-(2-pyridyl)-2-hexylamid

Die Titelverbindung wird aus den Verbindungen aus den Beispielen 3b und 17a nach dem in Beispiel la angegebenen Verfahren hergestellt.
MS (FAB): 766 (M+H)

**Beispiel 20**

N-[N-(2(S)-(4-Amino-1-piperidinyl-carbonyl-oxy)-3-phenyl-propionyl)-L-norvalinyl]-(2S,3R,4S)-1-cyclohexyl-3,4-dihydroxy-6-(2-pyridyl)-2-hexylamid-acetat

Die Titelverbindung wird aus der Verbindung aus Beispiel 19 nach dem in Beispiel 2 angegebenen Verfahren hergestellt. MS (FAB): 666 (M+H)

**Beispiel 21**

N-[N-(2(R)-Benzyl-3-(N-benzyl-piperidinyl-4-amino-carbonyl) propionyl)-L-norvalinyl]-(2S,3R,4S)-1-cyclohexyl-3,4-dihydroxy-6-(2-pyridyl)-2-hexylamid

Die Titelverbindung wird aus den Verbindungen aus den Beispielen 5b und 17a nach dem in Beispiel 1a angegebenen Verfahren hergestellt.
MS (FAB): 754 (M+H)

**Beispiel 22**

N-[N-(2(R)-Benzyl-3-(piperidinyl-4-amino-carbonyl)propionyl)-L-norvalinyl]-(2S,3R,4S)-1-cyclohexyl-3,4-dihydroxy-6-(2-pyridyl)-2-hexylamid-acetat

Die Titelverbindung wird aus der Verbindung aus Beispiel 21 nach dem in Beispiel 6 beschriebenen Verfahren hergestellt.
MS (FAB): 664 (M+H)

**Beispiel 23**

N-[N-(2(S)-(N-Benzyl-piperidinyl-4-amino-carbonyl-oxy)-3-phenyl-propionyl)-L-norvalinyl]-(2S,3R,4S)-1-cyclohexyl-3,4-dihydroxy-6-(2-pyridyl)-2-hexylamid

Die Titelverbindung wird aus den Verbindungen aus den Beispielen 7b und 17a nach dem in Beispiel 1a beschriebenen Verfahren hergestellt.
MS (FAB): 756 (M+H)

**Beispiel 24**

N-[N-(3-Phenyl-2(S)-(piperidinyl-4-amino-carbonyl-oxy)-propionyl)-L-norvalinyl]-(2S,3R,4S)-1-cyclohexyl-3,4-dihydroxy-6-(2-pyridyl)-2-hexylamid-acetat

Die Titelverbindung wird aus der Verbindung aus Beispiel 23 nach dem in Beispiel 6 angegebenen Verfahren hergestellt.

**Beispiel 25**

N-[N-(3-(4-(tert.-Butyloxycarbonyl)amino-1-piperidinyl-carbonyl)-2(R)-(1-naphthylmethyl)propionyl)-L-histidinyl]-(2S,3R,4S)-1-cyclohexyl-3,4-dihydroxy-6-(2-pyridyl)-2-hexylamid

Die Titelverbindung wird aus der Verbindung aus Beispiel 25a nach dem in Beispiel 1 angegebenen Verfahren hergestellt. MS (FAB): 852 (M+H)

a) N-[N-(3-(4-(tert.-Butyloxycarbonyl)amino-1-piperidinyl-carbonyl)-2(R)-(1-naphthylmethyl)propionyl)-L-histidinyl-(DNP)]-(2S,3R,4S)-1-cyclohexyl-3,4-dihydroxy-6-(2-pyridyl)-2-hexylamid

Die Titelverbindung wird aus den Verbindungen aus den Beispielen 1f und 25b nach dem in Beispiel 1a angegebenen Verfahren hergestellt.
MS (FAB): 1018 (M+H)

b) 3-[4-(tert.-Butyloxycarbonyl)amino-1-piperidinylcarbonyl]-2(R)-(1-naphthylmethyl)-propionsäure

Die Titelverbindung wird aus der Verbindung aus Beispiel 25c nach dem in Beispiel 1b angegebenen Verfahren hergestellt.
MS (DCI): 441 (M+H)

c) 3-[4-(tert.-Butyloxycarbonyl)amino-1-piperidinyl]-2(R)-(1-naphthylmethyl)-propionsäurebenzylester

Die Titelverbindung wird aus 2(R)-(Carboxymethyl)-3-(1-naphthyl)-propionsäurebenzylester (hergestellt nach J. Med. Chem. 31 (1988) 2277-2288) und der Verbindung aus Beispiel 1d nach dem in Beispiel 1c angegebenen Verfahren dargestellt.
MS (DCI): 531 (M+H)

**Beispiel 26**

N-[N-(3-(4-Amino-1-piperidinyl-carbonyl)-2(R)-(1-naphthyl-methyl)propionyl)-L-histidinyl]-(2S,3R,4S)-1-cyclohexyl-3,4-dihydroxy-6-(2-pyridyl)-2-hexylamid-acetat

Die Titelverbindung wird aus der Verbindung aus Beispiel 25 nach dem in Beispiel 2 angegebenen Verfahren hergestellt.
MS (FAB): 752 (M+H)

**Beispiel 27**

N-[N-(3-(N-Benzyl-piperidinyl-4-amino-carbonyl)-2(R)-(1-naphthylmethyl)-propionyl)-L-histidinyl]-(2S,3R,4S)-1-cyclohexyl-3,4-dihydroxy-6-(2-pyridyl)-2-hexylamid

Die Titelverbindung wird aus der Verbindung aus Beispiel 27a nach dem in Beispiel 1 angegebenen Verfahren hergestellt.
MS (FAB): 842 (M+H)

a) N-[N-(2(R)-(3-(N-Benzyl-piperidinyl-4-amino-carbonyl)-1-naphthylmethyl)-propionyl)-L-histidinyl(DNP)]-(2S,3R,4S)-1-cyclohexyl-3,4-dihydroxy-6-(2-pyridyl)-2-hexylamid

Die Titelverbindung wird aus den Verbindungen aus den Beispielen 1f und 27b nach dem in Beispiel la angegebenen Verfahren dargestellt.
MS (FAB): 1008 (M+H)

b) 2(R)-[3-(N-Benzyl-piperidinyl-4-amino-carbonyl)-1-naphthylmethyl]-propionsäure

Die Titelverbindung wird aus der Verbindung aus Beispiel 27c nach dem in Beispiel 5b angeführten Verfahren hergestellt.
MS (DCI): 431 (M+H)

c) 2(R)-[3-(N-Benzyl-piperidinyl-4-amino-carbonyl)-1-naphthylmethyl]-propionsäurebenzylester

Die Titelverbindung wird aus 4-Amino-N-benzylpiperidin und 2(R)-(Carboxymethyl)-3-(1-naphthyl)propionsäurebenzylester nach dem in Beispiel 1c angegebenen Verfahren hergestellt.
MS (DCI): 521 (M+H)

**Beispiel 28**

N-[N-(2(R)-(1-Naphthylmethyl)-3-(piperidinyl-4-amino-carbonyl)propionyl)-L-histidinyl]-(2S,3R,4S)-1-cyclohexyl3,4-dihydroxy-6-(2-pyridyl)-2-hexylamid-acetat
Die Titelverbindung wird aus der Verbindung aus Beispiel 27 nach dem in Beispiel 6 angegebenen Verfahren hergestellt.
MS (FAB): 752 (M+H)

**Beispiel 29**

N-[N-(2(S)-Benzyl-3-(4-tert.-butyloxycarbonyl)amino-1-piperidinyl-sulfonyl)-propionyl)-L-histidinyl]-(2S,3R,4S)-1-cyclohexyl-3,4-dihydroxy-6-(2-pyridyl)-2-hexylamid

Die Titelverbindung wird aus der Verbindung des Beispiels 29a analog der in Beispiel 1 beschriebenen Verfahrensweise hergestellt.
MS (FAB): 838 (M+H)

a) N-[N-(2(S)-Benzyl-3-(4-(tert.-butyloxycarbonyl)amino-1-piperidinyl-sulfonyl)-propionyl)-L-histidinyl(DNP)]-(2S,3R,4S)-1-cyclohexyl-3,4-dihydroxy-6-(2-pyridyl)-2-hexylamid

Die Titelverbindung wird z.B. nach dem Verfahren des Beispiels Ia aus 2(R,S)-Benzyl-3-[4-(tert.-butyloxycarbonyl) amino-1-piperidinyl-sulfonyl]-propionsäure hergestellt. Die beiden anfallenden Diastereomere werden durch Säulenchromatographie an Kieselgel mit dem Lösungsmittelgemisch aus Methylenchlorid und Methanol 96/4 bis 9/1 getrennt. Das zuerste eluierte Produkt hat einen optischen Drehwert von $[\alpha]_D^{20}$ = -35,9° (c=1, $CH_3OH$) und das nachfolgend eluierte Produkt einen Drehwert von $[\alpha]_D^{20}$ = -24,8° (c=1, $CH_3OH$). Einen der beiden Diastereomere ist die Titelverbindung.
MS (FAB): 1004 (M+H)

b) 2(R,S)-Benzyl)-3-[4-(tert.-butyloxycarbonyl)amino-1-piperidinyl-sulfonyl]-propionsäure

2-(R,S)-Benzyl-3-chlorsulfonyl-propionsäurebenzylester wird analog der EP-A 236 734 hergestellt. Anstelle des Benzylmalonsäuremonoethylesters wird der Benzylmalonsäuremonobenzylester eingesetzt. Zur Chlorsulfonylverbindung (2 mMol) in Methylenchlorid (3,2 ml) werden bei -10 °C 4-tert.-Butyloxycarbonyl-amino-piperidin (2 mMol) und Triethylamin (2 mMol), in 5,4 ml Methylenchlorid zugegeben. Nach Reaktion (15 min bei -15 °C) und Aufarbeitung werden 715 mg Sulfonamidderivat erhalten Fp: 115-117 °C.

Diese Substanz wird mit Pd/C in Methanol katalytisch bei Raumtemperatur hydriert und die Titelverbindung wird erhalten.
Fp: 161-163 °C
MS (FAB): 1004 (M+H)

**Beispiel 30**

N-[N-(3-(4-Amino-1-piperidinyl-sulfonyl)-2(R)-benzyl-propionyl)-L-histidinyl]-(2S,3R,4S)-1-cyclohexyl-3,4-dihydroxy-6-(2-pyridyl)-2-hexylamid-acetat

Die Titelverbindung wird aus der Verbindung aus Beispiel 29 nach dem Verfahren des Beispiels 2 hergestellt.
MS (FAB): 738 (M+H)

**Beispiel 31**

N-[N-(2(R)-Benzyl-3-(4-(tert.-butyloxycarbonyl)aminomethyl-1-piperidinylcarbonyl)-propionyl)-L-histidinyl]-(2S,3R,4S)-1-cyclohexyl-3,4-dihydroxy-6-(2-pyridyl)-2-hexylamid

Die Titelverbindung wird aus der Verbindung aus Beispiel 31a nach dem Verfahren des Beispiels 1 hergestellt.
MS (FAB): 816 (M+H)

a) N-[N-(2(R)-Benzyl-3-(4-(tert.-butyloxycarbonyl)amino-methyl-1-piperidinylcarbonyl)-propionyl)-L-histidinyl (DNP)]-(2S,3R,4S)-1-cyclohexyl-3,4-dihydroxy-6-(2-pyridyl)-2-hexylamid

Die Titelverbindung wird aus den Verbindungen aus den Beispielen 1f und 31b nach dem Verfahren des Beispiels Ia hergestellt.
MS (FAB): 982 (M+H)

b) 2(R)-Benzyl-3-[4-(tert.-butyloxycarbonyl)aminomethyl-1-piperidinylcarbonyl]-propionsäure

Die Titelverbindung wird aus der Verbindung aus Beispiel 31c nach dem in Beispiel 1b angegebenen Verfahren hergestellt.
MS (DCI): 405 (M+H)

c) 2(R)-Benzyl-3-[4-(tert.-butyloxycarbonyl)aminomethyl-1-piperidinylcarbonyl]-propionsäurebenzylester

Die Titelverbindung wird aus (2R)-2(Carboxymethyl)-3-phenylpropionsäurebenzylester und der Verbindung aus

Beispiel 31d nach dem im Beispiel 1c angeführten Verfahren hergestellt. MS (DCI): 495 (M+H)

d) 4-(tert.-Butyloxycarbonyl)aminomethyl-piperidin

10,8 g 4-(Aminomethyl)-piperidin werden in 100 ml $CH_2Cl_2$ gelöst, 25 g Di-tert.-butyldicarbonat zugefügt und die resultierende Lösung 3 Stunden bei Raumtemperatur gerührt. Nach Einengung wird der Rückstand in Diisopropylether aufgenommen, zweimal mit je 100 ml 5 %iger $NaHSO_4$-Lsg extrahiert, die vereinigten Extrakte mit $Na_2CO_3$ auf pH 9 eingestellt und dreimal mit 250 ml EE extrahiert. Trocknung über $Na_2SO_4$ und Einrotierung liefert 6 g der Titelverbindung als farbloses Öl.
$R_f$ ($SiO_2$, $CH_2Cl_2$/MeOH 9:1) : 0,12
MS (DCI): 215 (M+H)

**Beispiel 32**

N-[N-(3-(4-Aminomethyl-1-piperidinyl-carbonyl)-2(R)-benzylpropionyl)-L-histidinyl]-(2S,3R,4S)-1-cyclohexyl-3,4-dihydroxy-6-(2-pyridyl)-2-hexylamid-acetat

Diese Verbindung wird nach dem in Beispiel 2 angeführten Verfahren aus der Verbindung aus Beispiel 31 dargestellt; weißer, amorpher Feststoff.
MS (FAB): 716 (M+H)

**Beispiel 33**

N-[N-(2(S)-(4-(tert.-Butyloxycarbonyl)aminomethyl-1-piperidinylcarbonyl-oxy)-3-phenyl-propionyl)-L-histidinyl]-(2S,3R,4S)-1-cyclohexyl-3,4-dihydroxy-6-(2-pyridyl)-2hexylamid

Diese Verbindung wird aus der Verbindung aus Beispiel 33a nach dem in Beispiel 1 angegebenen Verfahren hergestellt.
MS (FAB): 818 (M+H)

a) N-[N-(2(S)-(4-tert.-Butyloxycarbonyl)aminomethyl-1piperidinylcarbonyl-oxy)-3-phenyl-propionyl)-L-histidinyl (DNF)]-(2S,3R,4S)-1-cyclohexyl-3,4-dihydroxy-6-(2-pyridyl)-2-hexylamid

Die Titelverbindung wird nach dem in Beispiel la angeführten Verfahren aus den Verbindungen aus den Beispielen 1f und 33b hergestellt; gelbes Harz.
MS (FAB): 984 (M+H)

b) 2(S)-[4-(tert.-Butyloxycarbonyl)aminomethyl-1piperidinyl-carbonyl-oxy]-3-phenyl-propionsäure

Diese Verbindung wird aus der Verbindung aus Beispiel 33c nach dem Verfahren des Beispiels 3b dargestellt.
MS (DCI): 407 (M+H)

c) 2(S)-[4-(tert.-Butyloxycarbonyl)aminomethyl-1piperidinyl-carbonyl-oxy]-3-phenyl-propionsäureethylester

Diese Verbindung wird aus 2(S)-Hydroxy-3-phenylpropionsäureethylester und der Verbindung aus Beispiel 31d nach dem in Beispiel 3c angeführten Verfahren hergestellt.
MS (DCI): 435 (M+H)

**Beispiel 34**

N-[N-(2(S)-(4-Aminomethyl-1-piperidinyl-carbonyl-oxy)-3-phenyl-propionyl)-L-histidinyl]-(2S,3R,4S)-1-cyclohexyl-3,4-dihydroxy-6-(2-pyridyl)-2-hexylamid-acetat.

Die Titelverbindung wird nach dem in Beispiel 2 angeführten Verfahren aus der Verbindung aus Beispiel 33 dargestellt; beiger, amorpher Feststoff.
MS (FAB): 718 (M+H)

**Beispiel 35**

N-[N-(2(R)-Benzyl-3-(4-(tert.-butyloxycarbonyl)amino-1-piperidinyl-carbonyl)-propionyl)-S-methyl-L-cysteinyl]-(2S,3R,4S)-1-cyclohexyl-3,4-dihydroxy-6-(2-pyridyl)-2hexylamid

90,0 mg der Verbindung aus Beispiel 35a werden in 1 ml $CH_2Cl_2$ mit 600 µl Trifluoressigsäure 4 h bei Raumtempartur gerührt. Es wird eingeengt, mit Toluol und $CH_2Cl_2$ codestilliert, der Rückstand in EE aufgenommen und mehrmals mit 1 N $NaHCO_3$-Lsg gewaschen. Nach Ausschütteln mit ges. NaCl-Lsg. und Trocknung über $Na_2SO_4$ wird eingeengt. Der resultierende Rückstand wird nach Trocknung im Hochvakuum in 3 ml Acetonitril gelöst, nacheinander 67 mg der Verbindung aus Beispiel 1b, 26 mg 1-Hydroxybenzotriazol, 32 µl N-Ethylmorpholin und 68 mg O-Benzotriazol-1-yl-N,N,N',N'-tetramethyluronium-hexafluorphosphat (HBTU) zugefügt und die Lösung 4 h bei Raumtemperatur gerührt. Die Lösung wurde mit 5 ml ges. NaCl-Lsg versetzt, dreimal mit EE extrahiert, die vereinten EE-Extrakte zweimal mit ges. $NaHCO_3$-Lösung und einmal mit ges. NaCl-Lösung gewaschen. Nach Trocknung über $Na_2SO_4$ und Einengung erfolgt Chromatographie an Kieselgel ($CH_2Cl_2$/MeOH 15:1). Es resultieren 80 mg der Titelverbindung als schwach beiges Harz.
$R_f$ ($SiO_2$, $CH_2Cl_2$/MeOH 9:1): 0,45
MS (FAB): 782 (M+H), 804 (M+Na)

a) S-Methyl-L-cystein-(2S,3R,4S)-1-cyclohexyl-3,4-dihydroxy-6-(2-pyridyl)-2-hexylamid

Diese Verbindung wird aus der Verbindung aus Beispiel 1g und BOC-S-Methyl-L-cystein nach dem im Beispiel 1f angegebenen Verfahren dargestellt.
MS (FAB): 510 (M+H), 516 (M+Li)

**Beispiel 36**

N-[N-(3-(4-Amino-1-piperidinyl-carbonyl-2(R)-benzyl-propionyl)-S-methyl-L-cysteinyl]-(2S,3R,4S)-1-cyclohexyl-3,4-dihydroxy-6-(2-pyridinyl)-2-hexylamid-acetat.

Die Titelverbindung wird aus der Verbindung aus Beispiel 35 nach dem in Beispiel 2 angeführten Verfahren hergestellt; blaßgelber, amorpher Feststoff.
MS (FAB): 682 (M+H), 704 (M+Na)

**Beispiel 37**

N-[N-(3-(4-tert.-Butyloxycarbonyl)aminomethyl-1-piperidinyl-carbonyl)-2(R)-(1-naphthylmethyl)propionyl)-L-histidinyl]-(2S,3R,4S)-1-cyclohexyl-3,4-dihydroxy-6-(2-pyridyl)-2hexylamid

Die Titelverbindung wird aus der Verbindung aus Beispiel 37a nach dem Verfahren des Beispiels 1 hergestellt.
MS (FAB): 866 (M+H)

a) N-[N-(3-(4-(tert.-Butyloxycarbonyl)aminomethyl-1-piperidinyl-carbonyl)-2(R)-(1-naphthylmethyl)propionyl)-L-histidinyl(DNP)]-(2S,3R,4S)-1-cyclohexyl-3,4-dihydroxy-6-(2pyridyl)-2-hexylamid

Diese Verbindung wird aus den Verbindungen aus den Beispielen 1f und 37b nach dem in Beispiel 1a angegebenen Verfahren dargestellt.
MS (FAB): 1032 (M+H), 1038 (M+Li)

b) 3-[4-(tert.-Butyloxycarbonyl)aminomethyl-1-piperidinylcarbonyl]-2(R)-(1-naphthylmethyl)-propionsäure

Diese Verbindung wird aus der Verbindung aus Beispiel 37c nach dem Verfahren des Beispiels 1b dargestellt.
MS (DCI): 455 (M+H)

c) 3-[4-(tert.-Butyloxycarbonyl)aminomethyl-1-piperidinylcarbonyl]-2(R)-(1-naphthylmethyl)-propionsäurebenzylester

Diese Verbindung wird aus 2(R)-(Carboxymethyl)-3-(1naphthyl)-propionsäurebenzylester und der Verbindung aus Beispiel 1d nach dem in Beispiel 1c angegebenen Verfahren dargestellt.
MS (DCI): 545 (M+H)

**Beispiel 38**

N-[N-(3-(4-Aminomethyl-1-piperidinyl-carbonyl)-2(R)-(1-naphthylmethyl)-propionyl)-L-histidinyl]-(2S,3R,4S)-1-cyclohexyl-3,4-dihydroxy-6-(2-pyridyl)-2-hexylamid-acetat

Die Titelverbindung wird aus der Verbindung aus Beispiel 37 nach dem in Beispiel 2 angegebenen Verfahren hergestellt.
MS (FAB): 766 (M+H)

**Beispiel 39**

N-[N-(3-(4-(tert.-Butyloxycarbonyl)amino-1-piperidinyl-carbonyl)-2(R)-(1-naphthylmethyl)propionyl)-S-methyl-L-cysteinyl]-(2S,3R,4S)-1-cyclohexyl-3,4-dihydroxy-6-(2-pyridyl)-2-hexylamid

Die Titelverbindung wird aus den Verbindungen der Beispiele 25b und 35a nach dem in Beispiel 35 angeführten Verfahren hergestellt.
MS (FAB): 832 (M+H)

**Beispiel 40**

N-[N-(3-(4-Amino-1-piperidinyl-carbonyl)-2(R)-(1-naphthylmethyl)-propionyl)-S-methyl-L-cysteinyl]-(2S,3R,4S)-1-cyclohexyl-3,4-dihydroxy-6-(2-pyridyl)-2-hexylamid-acetat

Die Titelverbindung wird aus der Verbindung aus Beispiel 39 nach dem Verfahren des Beispiels 2 dargestellt.
MS (FAB): 732 (M+H)

**Beispiel 41**

N-[N-(3-(4-(tert.-Butyloxycarbonyl)amino-1-piperidinyl-carbonyl)-2(R)-(1-tetrahydronaphthylmethyl)propionyl)-L-histidinyl]-(2S,3R,4S)-1-cyclohexyl-3,4-dihydro-6-(2-pyridyl-2-hexylamid

Die Titelverbindung wird aus der Verbindung aus Beispiel 41a nach dem Verfahren des Beispiels 1 hergestellt.
MS (FAB): 856 (M+H)

a) N-[N-(3-(4-(tert.-Butyloxycarbonyl)amino-1-piperidinyl-carbonyl)-2(R)-(1-tetrahydronaphthylmethyl)propionyl)-L-histidinyl(DNP)]-(2S,3R,4S)-1-cyclohexyl-3,4-dihydroxy-6-(2pyridyl)-2-hexylamid

Diese Verbindung wird aus den Verbindungen der Beispiele 1f und 41b nach dem in Beispiel 35 angegebenen Verfahren hergestellt.
MS (FAB): 1022 (M+H)

b) 3-[4-(tert.-Butyloxycarbonyl)amino-1-piperidinylcarbonyl]-2(R)-(1-tetrahydronaphthylmethyl)-propionsäure

Diese Verbindung entsteht neben der aus Beispiel 25b aus der Verbindung aus Beispiel 25c nach dem in Beispiel 1b angegebenen Verfahren, wenn die Hydrierzeit von 1h auf 24h verlängert wird. Die Isolierung der Titelverbindung erfolgt durch Chromatographie an KG ($CH_2Cl_2$ / MeOH 95 : 5).
MS (DCI): 445 (M+H)

**Beispiel 42**

N-[N-(3-(4-Amino-1-piperidinyl-carbonyl)-2(R)-(1-tetrahydronaphthylmethyl)propionyl)-L-histidinyl]-(2S,3R,4S) cyclohexyl-3,4-dihydroxy-6-(2-pyridyl)-2-hexylamid-acetat

Die Titelverbindung wird aus der Verbindung aus Beispiel 41 nach dem in Beispiel 2 angeführten Verfahren hergestellt.
MS (FAB): 756 (M+H)

**Beispiel 43**

N-[N-(2(R)-Benzyl-3-(4-(tert.-butyloxycarbonyl)amino-2,6-dimethyl-1-piperidinyl-carbonyl)-propionyl)-L-histidinyl]-(2S,3R,4S)-1-cyclohexyl-3,4-dihydroxy-6-(2-pyridyl)-2hexylamid

Die Titelverbindung wird aus der Verbindung aus Beispiel 43a nach dem in Beispiel 1 angegebenen Verfahren hergestellt.
MS (FAB): 830 (M+H)

a) N-[N-(2(R)-Benzyl-3-(4-(tert.-butyloxycarbonyl)amino-2,6-dimethyl-1-piperidinyl-carbonyl)-propionyl)-L-histidinyl (DNF)]-(2S,3R,4S)-1-cyclohexyl-3,4-dihydroxy-6-(2pyridyl)-2-hexylamid

Die Titelverbindung wird aus den Verbindungen aus den Beispielen 1f und 43b nach dem in Beispiel 35 angegebenen Verfahren hergestellt.
MS (FAB): 996 (M+H)

b) 2(R)-Benzyl-3-[4-(tert.-butyloxycarbonyl)amino-2,6dimethyl-1-piperidinyl-carbonyl]-propionsäure

Die Titelverbindung wird aus der Verbindung aus Beispiel 43c nach dem in Beispiel 1b angeführten Verfahren hergestellt.
MS (DCI): 419 (M+H)

c) 2(R)-Benzyl-3-[4-(tert.butyloxycarbonyl)amino-2,6dimethyl-1-piperidinyl-carbonyl]-propionsäurebenzylester

Die Titelverbindung wird aus (2R)-2-(Carboxymethyl)-3-phenylpropionsäurebenzylester und der Verbindung aus Beispiel 43d nach dem Verfahren des Beispiels 1c dargestellt.
MS (DCI): 509 (M+H)

d) 4-(tert.-Butyloxycarbonyl)amino-2,6-dimethyl-piperidin

Diese Verbindung wird aus der Verbindung aus Beispiel 43c nach dem Verfahren des Beispiels 1d hergestellt.
MS (DCI): 229 (M+H)

e) 1-Benzyl-4-[(tert.-butyloxycarbonyl)amino]-2,6-dimethyl-piperidin

Diese Verbindung wird aus der Verbindung aus Beispiel 43f nach dem in Beispiel 1e angeführten Verfahren hergestellt.
MS (DCI): 319 (M+H)

f) 4-Amino-1-benzyl-2,6-dimethyl-piperidin

5 g der Verbindung aus Beispiel 43g werden in 10 ml Methanol gelöst, 2 ml konz. Ammoniak-Lsg. und Raney-Nickel zugefügt und im Autoklaven bei einem Druck von 100 atm hydriert. Es wird filtriert, eingeengt und der erhaltene Rückstand durch Destillation gereinigt.
MS (DCI): 219 (M+H)

g) 1-Benzyl-2,6-dimethyl-4-piperidonoxim

10 g 1-Benzyl-2,6-dimethyl-4-piperidon (hergestellt aus Benzylamin und Crotonsäureethylester analog zu Bull. Chem. Soc. Japan 31 (1958)418) werden in 60 ml Methanol gelöst und diese Lösung zu einer Lösung von 3,8 g Hydroxylamin-Hydrochlorid und 4,5 g Natriumacetat in 150 ml Wasser getropft. Man rührt 2h bei 60°C, kühlt auf 0°C und saugt das abgeschiedene Oxim ab. Nach Trocknung resultieren 5,2 g der Titelverbindung.
MS (DCI): 233 (M+H)

**Beispiel 44**

N-[N-(3-(4-Amino-2,6-dimethyl-1-piperidinyl-carbonyl)-2(R)-benzyl-propionyl)-L-histidinyl]-(2S,3R,4S)-1-cyclohexyl-3,4dihydroxy-6-(2-pyridyl)-2-hexylamid-acetat

Die Titelverbindung wird aus der Verbindung aus Beispiel 43 nach dem in Beispiel 2 angeführten Verfahren hergestellt.
MS (FAB): 730 (M+H)

**Beispiel 45**

N-[N-(2(S)-(4-(tert.-Butyloxycarbonyl)amino-2,6-dimethyl-1-piperidinyl-carbonyl-oxy)-3-phenyl-propionyl)-L-histidinyl]-(2S,3R,4S)-1-cyclohexyl-3,4-dihydroxy-6-(2-pyridyl)-2hexylamid

Die Titelverbindung wird aus der Verbindung aus Beispiel 45a nach dem in Beispiel 1 angegebenen Verfahren hergestellt.
MS (FAB): 832 (M+H)

a) N-[N-(2(S)-(4-(tert.-Butyloxycarbonyl)amino-2,6-dimethyl-1-piperidinyl-carbonyl-oxy)-3-phenyl-propionyl)-L-histidinyl(DNP)]-(2S,3R,4S)-1-cyclohexyl-3,4-dihydroxy-6-(2pyridyl)-2-hexylamid

Die Titelverbindung wird aus den Verbindungen aus den Beispielen 1f und 45b nach dem in Beispiel 35 angegebenen Verfahren hergestellt.
MS (FAB): 998 (M+H)

b) 2(S)-[4-(tert.-Butyloxycarbonyl)amino-2,6-dimethyl-1piperidinyl-carbonyl-oxy]-3-phenyl-propionsäure

Diese Verbindung wird aus der Verbindung aus Beispiel 45c nach dem Verfahren des Beispiels 3b hergestellt.
MS (DCI): 421 (M+H)

c) 2(S)-[4-(tert.-Butyloxycarbonyl)amino-2,6-dimethyl-1piperidinyl-carbonyl-oxy]-3-phenyl-propionsäureethylester

Diese Verbindung wird aus 2(S)-Hydroxy-3-phenylpropionsäureethylester und der Verbindung aus Beispiel 43d nach dem Verfahren des Beispiels 3c hergestellt.
MS (DCI): 449 (M+H)

**Beispiel 46**

N-[N-(2(S)-(4-Amino-2,6-dimethyl-1-piperidinyl-carbonyl-oxy)-3-phenyl-propionyl)-L-histidinyl]-(2S,3R,4S)-1-cyclohexyl-3,4dihydroxy-6-(2-pyridyl)-2-hexylamid-acetat

Die Titelverbindung wird aus der Verbindung aus Beispiel 45 nach dem in Beispiel 2 angegebenen Verfahren hergestellt.
MS (FAB): 732 (M+H)

**Patentansprüche**

**Patentansprüche für folgende Vertragsstaaten : AT, BE, CH, DE, DK, FR, GB, IT, LI, LU, NL, SE**

**1.** Verbindung der Formel I

$$R^1 - X - Y - CHR^2 - CO - B - NH - CHR^3 - CH(OH) - CHR^4 - R^5 \qquad (I)$$

in welcher

$R^1$ einen Rest der Formel II, III oder IV

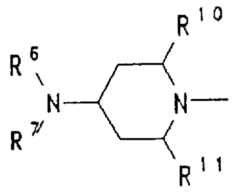

(II)

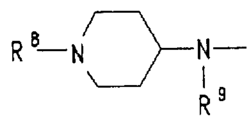

(III)

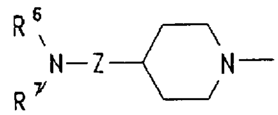

(IV)

bedeutet, worin

$R^6$ für Wasserstoff oder tert. Butyloxycarbonyl steht,
$R^7$ Wasserstoff bedeutet,
$R^8$ Wasserstoff oder Benzyl bedeutet,
$R^9$ Wasserstoff bedeutet,
$R^{10}$ und $R^{11}$ zusammen Wasserstoff oder Methyl bedeuten, und
Z $-CH_2-$ ist,
X -CO- oder $-SO_2-$ ist,
Y $-CH_2-$ oder -O- bedeutet,
$R^2$ Benzyl oder 1- oder 2-Naphthylmethyl bedeutet,
$R^3$ Cyclohexylmethyl bedeutet,
$R^4$ Hydroxy ist,
$R^5$ für einen Rest der Formel V

$$(CH_2)_s - CHR^{15} - Het \qquad (V)$$

steht, worin

$R^{15}$ Wasserstoff bedeutet,
Het für einen 2-Pyridyl-Rest steht, und
s 1 bedeutet, und

B einen Rest einer Aminosäure H-B-OH aus der Reihe Histidin, Norvalin, oder S-Methylcystein bedeutet,
oder deren physiologisch verträgliche Salze.

**2.** Verbindung der Formel I gemäß Anspruch 1, dadurch gekennzeichnet, daß $R^{10}$ und $R^{11}$ Wasserstoff bedeuten und X für -CO- steht.

**3.** Verbindung der Formel I aus der Reihe N-[N-(3-(4-(tert.-Butyloxycarbonyl)amino-1-piperidinyl-carbonyl)-2(R)-(1-tetrahydronaphthylmethyl)propionyl)-L-histidinyl]-(2S,3R,4S)1-cyclohexyl-3,4-dihydroxy-6-(2-pyridyl)-2-hexylamid und N-[N-(3-(4-Amino-1-piperidinylcarbonyl)-2(R)-(1-tetrahydronaphthylmethyl)propionyl)-L-histidinyl]-(2S,3R,4S)-cyclohexyl-3,4-dihydroxy-6-(2-pyridyl)-2-hexylamid-acetat.

**4.** N-[N-(3-(4-Amino-1-piperidinyl-carbonyl)-2(R)-benzylpropionyl)-Lhistidinyl]-(2S,3R,4S)-cyclohexyl-3,4-dihydroxy-6-(2-pyridyl)-2-hexylamid-acetat.

**5.** Verfahren zur Herstellung einer Verbindung der Formel I gemäß den Ansprüchen 1 bis 4, dadurch gekennzeichnet, daß man ein Fragment mit endständiger Carboxylgruppe oder dessen reaktives Derviat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**6.** Verbindung der Formel I gemäß einem der Ansprüche 1 bis 4 zur Anwendung als Heilmittel.

7. Pharmazeutische Zubereitung enthaltend mindestens eine Verbindung der Formel I gemäß einem der Ansprüche 1 bis 4.

8. Verfahren zur Herstellung einer Zubereitung gemäß Anspruch 7, dadurch gekennzeichnet, daß man eine Verbindung der Formel I oder deren physiologisch verträgliches Salz mit den geeigneten Zusatzstoffen in eine geeignete Darreichungsform bringt.

**Patentansprüche für folgende Vertragsstaaten : ES, GR**

1. Verfahren zur Herstellung einer Verbindung der Formel I

$$R^1 - X - Y - CHR^2 - CO - B - NH - CHR^3 - CH(OH) - CHR^4 - R^5 \qquad (I)$$

in welcher

$R^1$ einen Rest der Formel II, III oder IV

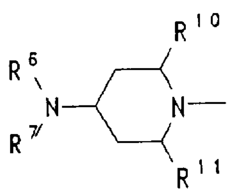

( I I )

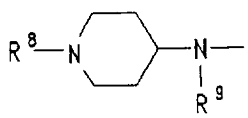

( I I I )

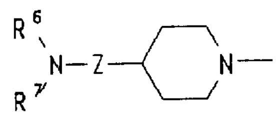

( I V )

bedeutet, worin

| | |
|---|---|
| $R^6$ | für Wasserstoff oder tert. Butyloxycarbonyl steht, |
| $R^7$ | Wasserstoff bedeutet, |
| $R^8$ | Wasserstoff oder Benzyl bedeutet, |
| $R^9$ | Wasserstoff bedeutet, |
| $R^{10}$ und $R^{11}$ | zusammen Wasserstoff oder Methyl bedeuten, und |
| Z | $-CH_2-$ ist, |
| X | -CO- oder-$SO_2$- ist, |
| Y | $-CH_2-$ oder -O- bedeutet, |
| $R^2$ | Benzyl oder 1- oder 2-Naphthylmethyl bedeutet, |
| $R^3$ | Cyclohexylmethyl bedeutet, |
| $R^4$ | Hydroxy ist, |
| $R^5$ | für einen Rest der Formel V |

$$(CH_2)_s - CHR^{15} - Het \qquad (V)$$

steht, worin

$R^{15}$ Wasserstoff bedeutet,
Het für einen 2-Pyridyl-Rest steht, und
s 1 bedeutet, und

B einen Rest einer Aminosäure H-B-OH aus der Reihe Histidin, Norvalin, oder S-Methylcystein bedeutet,
oder deren physiologisch verträgliche Salze,

dadurch gekennzeichnet, daß man ein Fragment mit endständiger Carboxylgruppe oder dessen reaktives Derviat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

**2.** Verfahren gemäß Anspruch 1, dadurch gekennzeichnet, daß man eine Verbindung der Formel I herstellt, worin $R^{10}$ und $R^{11}$ Wasserstoff bedeuten und X für -CO- steht.

**3.** Verfahren gemäß Anspruch 1, dadurch gkennzeichnet, daß man eine Verbindung der Formel I aus der Reihe N-[N-(3-(4-(tert.-Butyloxycarbonyl)amino-1-piperidinyl-carbonyl)-2(R)-(1-tetrahydronaphthylmethyl)propionyl)-L-histidinyl]-(2S,3R,4S)-1 cyclohexyl-3,4-dihydroxy-6-(2-pyridyl)-2-hexylamid und N-[N-(3-(4-Amino-1-piperidinyl-carbonyl)-2(R)-(1-tetrahydronaphthylmethyl)propionyl)-Lhistidinyl]-(2S,3R,4S)-cyclohexyl-3,4-dihydroxy-6-(2-pyridyl)-2-hexylamid-acetat herstellt.

**4.** Verfahren gemäß Anspruch 1, dadurch gekennzeichnet, daß man N-[N-(3-(4-Amino-1-piperidinyl-carbonyl)-2(R)-benzylpropionyl)-L-histidinyl]-(2S,3R,4S)-cyclohexyl-3,4-dihydroxy-6-(2-pyridyl)-2-hexylamid-acetat herstellt.

**5.** Verfahren zur Herstellung einer Zubereitung enthaltend mindestens eine Verbindung der Formel I gemäß einem der Ansprüche 1 bis 4, dadurch gekennzeichnet, daß man diese oder deren physiologisch verträgliches Salz mit den geeigneten Zusatzstoffen in eine geeignete Darreichungsform bringt.

**Claims**

**Claims for the following Contracting States : AT, BE, CH, DE, DK, FR, GB, IT, LI, LU, NL, SE**

**1.** A process for the preparation of a compound of the formula I

$$R^1\text{-X-Y-CHR}^2\text{-CO-B-NH-CHR}^3\text{-CH(OH)-CHR}^4\text{-R}^5 \quad (I)$$

in which

$R^1$ is a radical of the formula II, III or IV

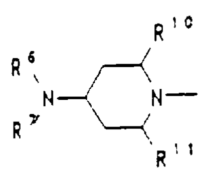

(II)

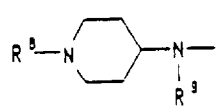

(III)

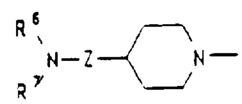

(IV)

in which

- $R^6$ is hydrogen or tert-butyloxycarbonyl,
- $R^7$ is hydrogen,
- $R^8$ is hydrogen or benzyl,
- $R^9$ is hydrogen,
- $R^{10}$ and $R^{11}$ together are hydrogen or methyl, and
- Z is $-CH_2-$,
- X is -CO- or $-SO_2-$,
- Y is $-CH_2-$ or -O-,
- $R^2$ is benzyl or 1- or 2-naphthylmethyl,
- $R^3$ is cyclohexylmethyl,
- $R^4$ is hydroxyl,
- $R^5$ is a radical of the formula V

$$(CH_2)_s\text{ - CHR}^{15}\text{ - Het} \quad (V)$$

in which

$R^{15}$ is hydrogen,
Het is a 2-pyridyl radical, and
s is 1, and

B is a residue of an amino acid H-B-OH from the series of histidine, norvaline or S-methylcysteine, or the physiologically tolerated salts thereof.

**2.** A compound of the formula I as claimed in claim 1, wherein $R^{10}$ and $R^{11}$ are hydrogen, and X is -CO-.

**3.** A compound of the formula I from the series of N-[N-(3-(4-(tert-butyloxycarbonyl)amino-1-piperidinyl-carbonyl)-2(R)-(1-tetrahydronaphthylmethyl)propionyl)-L-histidinyl]-(2S,3R,4S)-1-cyclohexyl-3,4-dihydroxy-6-(2-pyridyl)-2-hexylamideandN-[N-(3-(4-amino-1-piperidinylcarbonyl)-2(R)-(1-tetrahydronaphthylmethyl)propionyl)-L-histidinyl]-(2S,3R,4S)-cyclohexyl-3,4-dihydroxy-6-(2pyridyl)-2-hexylamide acetate.

**4.** N-[N-(3-(4-Amino-1-piperidinyl-carbonyl)-2(R)-benzylpropionyl)-L-histidinyl]-(2S,3R,4S)-cyclohexyl-3,4dihydroxy-6-(2-pyridyl)-2-hexylamide acetate.

**5.** A process for the preparation of a compound of the formula I as claimed in claims 1 to 4, which comprises coupling a fragment with a terminal carboxyl group or a reactive derivative thereof to an appropriate fragment with a free amino group, where appropriate eliminating (a) protective group(s) temporarily introduced to protect other functional groups, and converting the compound obtained in this way, where appropriate, into the physiologically tolerated salt thereof.

**6.** A compound of the formula I as claimed in any of claims 1 to 4 for use as medicine.

**7.** A pharmaceutical composition comprising at least one compound of the formula I as claimed in any of claims 1 to 4.

**8.** A process for the preparation of a composition as claimed in claim 7, which comprises converting a compound of the formula I or the physiologically tolerated salt thereof with the suitable additives into a suitable dosage form.

**Claims for the following Contracting States : ES, GR**

**1.** A compound of the formula I

$$R^1\text{-X-Y-CHR}^2\text{-CO-B-NH-CHR}^3\text{-CH(OH)-CHR}^4\text{-R}^5 \quad (I)$$

in which

$R^1$ is a radical of the formula II, III or IV

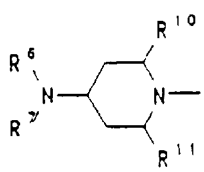
(II)

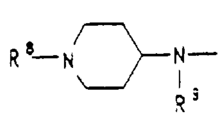
(III)

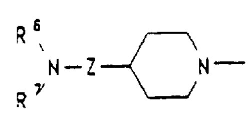
(IV)

in which
$R^6$ is hydrogen or tert-butyloxycarbonyl,
$R^7$ is hydrogen,
$R^8$ is hydrogen or benzyl,
$R_9$ hydrogen,
$R^{10}$ and $R^{11}$ together are hydrogen or methyl, and

Z is $-CH_2-$,
X is -CO- or $-SO_2-$,
Y is $-CH_2-$ or -O-,
$R^2$ is benzyl or 1- or 2-naphthylmethyl,
$R^3$ is cyclohexylmethyl,
$R^4$ is hydroxyl,
$R^5$ is a radical of the formula V

$$(CH_2)_s\text{-}CHR^{15}\text{-Het} \qquad (V)$$

in which

$R^{15}$ is hydrogen,
Het is a 2-pyridyl radical, and
s is 1, and

B is a residue of an amino acid H-B-OH from the series

of histidine, norvaline or S-methylcysteine, or the physiologically tolerated salts thereof, which comprises coupling a fragment with a terminal carboxyl group or a reactive derivative thereof to an appropriate fragment with a free amino group, where appropriate eliminating (a) protective group(s) temporarily introduced to protect other functional groups, and converting the compound obtained in this way, where appropriate, into the physiologically tolerated salt thereof.

**2.** The process as claimed in claim 1, wherein a compound of the formula I in which $R^{10}$ and $R^{11}$ are hydrogen, and X is -CO-, is prepared.

**3.** The process as claimed in claim 1, wherein a compound of the formula I from the series of N-[N-(3-(4-(tert-butyloxycarbonyl)amino-1-piperidinyl-carbonyl)-2(R)-(1-tetrahydronaphthylmethyl)propionyl)-L-histidinyl]-(2S,3R,4S)-1-cyclohexyl-3,4-dihydroxy-6-(2-pyridyl)-2-hexylamide and N-[N-(3-(4-amino-1-piperidinylcarbonyl)-2(R)-(1-tetrahydronaphthylmethyl)propionyl)-L-histidinyl]-(2S,3R,4S)-cyclohexyl-3,4-dihydroxy-6-(2-pyridyl)-2-hexylamide acetate is prepared.

**4.** The process as claimed in claim 1, wherein N-[N-(3-(4-amino-1-piperidinyl-carbonyl)-2(R)-benzylpropionyl)-L-histidinyl]-(2S,3R,4S)-cyclohexyl-3,4-dihydroxy-6-(2-pyridyl)-2-hexylamide acetate is prepared.

**5.** A process for the preparation of a composition comprising at least one compound of the formula I as claimed in any of claims 1 to 4, which comprises converting the latter or the physiologically tolerated salt thereof with the suitable additives into a suitable dosage form.

## Revendications

### Revendications pour les Etats contractants suivants : AT, BE, CH, DE, DK, FR, GB, IT, LI, LU, NL, SE

**1.** Composé de formule I

$$R^1\text{-X-Y-CHR}^2\text{-CO-B-NH-CHR}^3\text{-CH(OH)-CHR}^4\text{-R}^5 \qquad (I)$$

dans laquelle

$R^1$ représente un radical de formule II, III ou IV

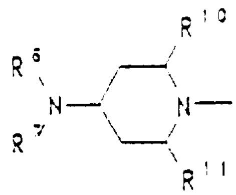

( I I )

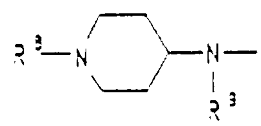

( I I I )

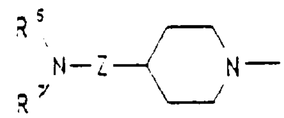

( I V )

dans lesquelles

| | |
|---|---|
| $R^6$ | représente un atome d'hydrogène ou un groupe tert.-butyloxycarbonyle, |
| $R^7$ | représente un atome d'hydrogène, |
| $R^8$ | représente un atome d'hydrogène ou un groupe benzyle, |
| $R^9$ | représente un atome d'hydrogène, |
| $R^{10}$ et $R^{11}$ | représentent chacun un atome d'hydrogène ou un groupe méthyle, et |
| Z | représente $-CH_2-$, |
| X | représente -CO- ou $-SO_2-$, |
| Y | représente $-CH_2-$ ou -O-, |
| $R^2$ | représente un groupe benzyle ou un groupe 1- ou 2-naphtylméthyle, |
| $R^3$ | représente un groupe cyclohexylméthyle, |
| $R^4$ | représente un groupe hydroxy, |
| $R^5$ | représente un groupe de formule V |

$$(CH_2)_s\text{-}CHR^{15}\text{-Het} \qquad (V)$$

dans laquelle $R^{15}$ représente un atome d'hydrogène,
Het représente un groupe 2-pyridyle, et
s vaut 1, et

| | |
|---|---|
| B | représente un résidu d'un aminoacide H-B-OH choisi parmi l'histidine, la norvaline et la S-méthylcystéine, |

ou un de ses sels physiologiquement acceptables.

**2.** Composé de formule I selon la revendication 1, caractérisé en ce que $R^{10}$ et $R^{11}$ représentent un atome d'hydrogène et X représente -CO-.

**3.** Composé de formule I choisi parmi le N-[N-(3-(4-(tert.butyloxycarbonyl)amino-1-pipéridinyl-carbonyl)-2(R)-(1-tétrahydronaphtylméthyl)propionyl)-L-histidinyl]-(2S,3R,4S)-1-cyclohexyl-3,4-dihydroxy-6-(2-pyridyl)-2-hexylamide et l'acétate de N-[N-(3-(4-amino-1-pipéridinyl-carbonyl)-2(R)-(1-tétrahydronaphtylméthyl)-propionyl)-L-histidinyl]-(2S,3R,4S)-cyclohexyl-3,4-dihydroxy-6-(2-pyridyl)-2-hexylamide.

**4.** Acétate de N-[N-(3-(4-amino-1-pipéridinyl-carbonyl)-2(R)-benzylpropionyl)-L-histidinyl]-(2S,3R,4S)-cyclohexyl-3,4-dihydroxy-6-(2-pyridyl)-2-hexylamide.

**5.** Procédé pour la fabrication d'un composé de formule I selon les revendications 1 à 4, caractérisé en ce que l'on couple un fragment ayant un groupe carboxy terminal ou un de ses dérivés réactifs avec un fragment correspondant ayant un groupe amino libre, on élimine un (des) groupe(s) protecteur(s) éventuellement introduit(s) de manière temporaire pour la protection d'autres groupes fonctionnels et l'on convertit éventuellement le composé ainsi obtenu en sel physiologiquement acceptable.

**6.** Composé de formule I selon l'une des revendications 1 à 4, pour une utilisation en tant que médicament.

**7.** Composition pharmaceutique comprenant au moins un composé de formule I selon l'une des revendications la 4.

**8.** Procédé de préparation d'une composition selon la revendication 7, caractérisé en ce que l'on met un composé de

formule I ou un de ses sels physiologiquement acceptables, avec les additifs appropriés, sous une forme d'administration appropriée.

**Revendications pour les Etats contractants suivants : ES, GR**

1. Procédé pour la préparation d'un composé de formule I

$$R^1\text{-X-Y-CHR}^2\text{-CO-B-NH-CHR}^3\text{-CH(OH)-CHR}^4\text{-R}^5 \qquad (I)$$

dans laquelle

$R^1$ représente un radical de formule II, III ou IV

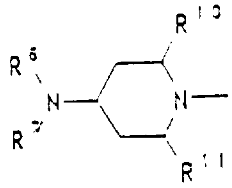
( I I )

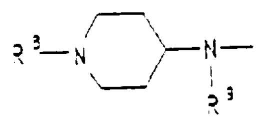
( I I I )

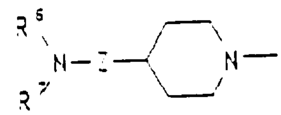
( I V )

dans lesquelles
$R^6$ représente un atome d'hydrogène ou un groupe tert.-butyloxycarbonyle,
$R^7$ représente un atome d'hydrogène,
$R^8$ représente un atome d'hydrogène ou un groupe benzyle,
$R^9$ représente un atome d'hydrogène,
$R^{10}$ et $R^{11}$ représentent chacun un atome d'hydrogène ou un groupe méthyle, et
Z représente $-CH_2-$,
X représente -CO- ou $-SO_2-$,
Y représente $-CH_2-$ ou -O-,
$R^2$ représente un groupe benzyle ou un groupe 1- ou 2-naphtylméthyle,
$R^3$ représente un groupe cyclohexylméthyle,
$R^4$ représente un groupe hydroxy,
$R^5$ représente un groupe de formule V

$$(CH_2)_s\text{-CHR}^{15}\text{-Het} \qquad (V)$$

dans laquelle $R^{15}$ représente un atome d'hydrogène,
Het représente un groupe 2-pyridyle, et
s vaut 1, et

B représente un résidu d'un aminoacide H-B-OH choisi parmi l'histidine, la norvaline et la S-méthylcys-téine,.

ou d'un de ses sels physiologiquement acceptable,
caractérisé en ce que l'on couple un fragment ayant un groupe carboxy terminal ou un de ses dérivés réactifs avec un fragment correspondant ayant un groupe amino libre, on élimine un (des) groupe(s) protecteur(s) éventuellement introduit(s) de manière temporaire pour la protection d'autres groupes fonctionnels et l'on convertit éventuellement le composé ainsi obtenu en sel physiologiquement acceptable

2. Procédé selon la revendication 1, caractérisé en ce que $R^{10}$ et $R^{11}$ représentent un atome d'hydrogène et X représente -CO-.

3. Procédé selon la revendication 1, caractérisé en ce que l'on prépare un composé de formule I choisi parmi le N-[N-(3-(4-(tert.butyloxycarbonyl)amino-1-pipéridinyl-carbonyl)-2(R)-(1-tétrahydronaphtylméthyl)-propionyl)-L-histidinyl]-(2S,3R,4S)-1-cyclohexyl-3,4-dihydroxy-6-(2-pyridyl)-2-hexylamide et l'acétate de N-[N-(3-(4-amino-1-pipéridinyl-carbonyl)-2(R)-(1-tétrahydronaphtylméthyl)propionyl)-L-histidinyl]-(2S,3R,4S)-cyclohexyl-3,4-dihydroxy-6-(2-pyridyl)-2-hexylamide.

**4.** Procédé selon la revendication 1, caractérisé en ce que l'on prépare l'acétate de N-[N-(3-(4-amino-1-pipéridinyl-carbonyl)-2(R)-benzylpropionyl)-L-histidinyl]-(2S,3R,4S)-cyclohexyl-3,4-dihydroxy-6-(2-pyridyl)-2-hexylamide.

**5.** Procédé de préparation d'une composition comprenant au moins un composé de formule I selon l'une des revendications 1 à 4, caractérisé en ce que l'on met ce composé ou un de ses sels physiologiquement acceptables, avec les additifs appropriés, sous une forme d'administration appropriée.